# EUROPEAN PATENT SPECIFICATION

(11) **EP 2 184 294 B1**
(45) Date of publication and mention of the grant of the patent: **26.02.2014**
(21) Application number: 09166163.7
(22) Date of filing: 23.07.2009
(51) Int. Cl.: C07K 14/395, C12N 1/18, C12P 7/06

(54) **Nucleic acids and constructs for increasing galactose catabolism and methods therefor**
Nukleinsäuren und Konstrukte zur Erhöhung des Galaktosekatabolismus und Verfahren dafür
Acides nucléiques et constructions pour augmenter le catabolisme du galactose et procédés correspondants

(30) Priority: 30.10.2008 KR 20080107277
(43) Date of publication of application: 12.05.2010
(73) Proprietor: Samsung Electronics Co., Ltd., Suwon-si, Gyeonggi-do, 443-742 (KR)
(72) Inventor: Yu, Byung Jo, Gyeonggi-do (KR); Park, Jae Chan, Gyeonggi-do (KR); Koo, Hyun Min, Seoul (KR); Jin, Yong Su, Gyeonggi-do (KR); Lee, Ki Sung, Gyeonggi-do (KR)
(74) Representative: Grünecker, Kinkeldey, Stockmair & Schwanhäusser

(56) References cited:
- ZHANG ZHIZHOU ET AL: "Functional dissection of the global repressor Tup1 in yeast: Dominant role of the C-terminal repression domain" GENETICS, vol. 161, no. 3, July 2002 (2002-07), pages 957-969, XP002574285 ISSN: 0016-6731
- TZAMARIAS AND K STRUHL D: "Functional dissection of the yeast cyc8-tup1 transcriptional co-repressor complex" NATURE, NATURE PUBLISHING GROUP, LONDON, UK, vol. 369, 30 June 1994 (1994-06-30), pages 758-761, XP002086762 ISSN: 0028-0836
- KOMACHI KELLY ET AL: "Residues in the WD repeats of Tup1 required for interaction with alpha-2" MOLECULAR AND CELLULAR BIOLOGY, vol. 17, no. 10, 1997, pages 6023-6028, XP002574286 ISSN: 0270-7306
- SPRAGUE ELIZABETH R ET AL: "Structure of the C-terminal domain of Tup1, a corepressor of transcription in yeast" EMBO (EUROPEAN MOLECULAR BIOLOGY ORGANIZATION) JOURNAL, vol. 19, no. 12, 15 June 2000 (2000-06-15) , pages 3016-3027, XP002574287 ISSN: 0261-4189
- JABET CAROLE ET AL: "Characterization of the N-terminal domain of the yeast transcriptional repressor Tup1: Proposal for an association model of the repressor complex Tup1cntdotSsn6" JOURNAL OF BIOLOGICAL CHEMISTRY, vol. 275, no. 12, 24 March 2000 (2000-03-24), pages 9011-9018, XP002574288 ISSN: 0021-9258
- CARRICO PAULINE M ET AL: "Mutational analysis of the Tup1 general repressor of yeast" GENETICS, vol. 148, no. 2, February 1998 (1998-02), pages 637-644, XP002574289 ISSN: 0016-6731

## Description

### BACKGROUND

### 1. Field

This application relates to a recombinant gene associated with increased galactose catabolism, a recombinant vector and microorganism containing the same, and to methods of making and using these compositions. More particularly, this application relates to a recombinant gene repressing expression of a gene involved in galactose catabolism, in which all or a part of a repression domain is inactivated.

### 2. Description of the Related Art

With the globally increasing concern about exhaustion of resources and pollution of the environment due to overuse of fossil fuels, development of new and renewable alternative energy sources for stable and continuous production of energy is being considered. Among such alternative energy resources under development, technology for producing energy from biomass has been receiving considerable attention.

In recent times, there has been considerable interest in the prospect of using algae as a source of biomass. An advantage of algae is its abundance and rapid growth. Also, since algae consume carbon dioxide and exhaust oxygen for growth, they offer a potential solution to both energy production and pollution concerns. However, algae have not yet been produced on a large scale for use in a variety of applications. Additionally, hydrolysates of biomass derived from algae contain a large amount of galactose. Accordingly, effective use of the abundant galactose in such hydrolysates is the first step toward developing a biological process for converting hydrolysates of algae-derived biomass into useful materials by fermenting organisms, such as yeast.

However, although galactose can be catabolized by naturally occurring microorganisms, such as yeast, the uptake and metabolic utilization rate of galactose is much lower than that of glucose.

ZHANG ZHIZOU ET AL: "Functional dissection of the global repressor Tup1 in yeast: Dominant role of the C-terminal repression domain", GENETICS, vol. 161, no. 3, July 2002, pages 957-969 relates to functional dissection of the global repressor Tup1 in yeast. This paper investigates the effect of mutations of the Tup1 gene with respect to their effect on the function of the Tup1 protein that is known to function, in association with Cyc8 (Ssn6), as a general repressor of transcription. The authors conclude that the C-terminal repression domain of the Tup1 protein, which is located near the beginning of the WD repeat domain, plays a dominant role in repression.

### SUMMARY

Disclosed herein is a novel recombinant gene increasing galactose catabolism when overexpressed, and a method of increasing volumetric productivity of ethanol from a carbon source containing galactose by using yeast, or other fermenting microorganism, transformed with the recombinant gene.

Disclosed herein is an isolated polynucleotide. In an embodiment, the isolated polynucleotide has a nucleotide sequence encoding a recombinant polypeptide, the amino acid sequence of which consists of SEQ ID NO:2.

Also disclosed herein is a recombinant polypeptide. In an embodiment, the isolated polypeptide has the amino acid sequence of SEQ ID NO:2.

In another embodiment, a recombinant vector including the isolated polynucleotide is disclosed.

Also disclosed is a recombinant microorganism including the isolated polynucleotide is disclosed.

Also disclosed is a method of producing the recombinant microorganism. In an embodiment, the method includes transforming a yeast with the recombinant vector including the isolated polynucleotide.

Also disclosed herein is a method of producing ethanol from a galactose-containing carbon source. In an embodiment, the method includes culturing a recombinant yeast disclosed herein in a galactose-containing carbon source such that ethanol is produced, wherein production of ethanol is increased by overexpression of the isolated polynucleotide.

These and other embodiments, advantages and features of the invention become clear when detailed description and examples are provided in subsequent sections.

### BRIEF DESCRIPTION OF THE DRAWINGS

Exemplary embodiments are described in further detail below with reference to the accompanying drawings. It should be understood that various aspects of the drawings may have been exaggerated for clarity.

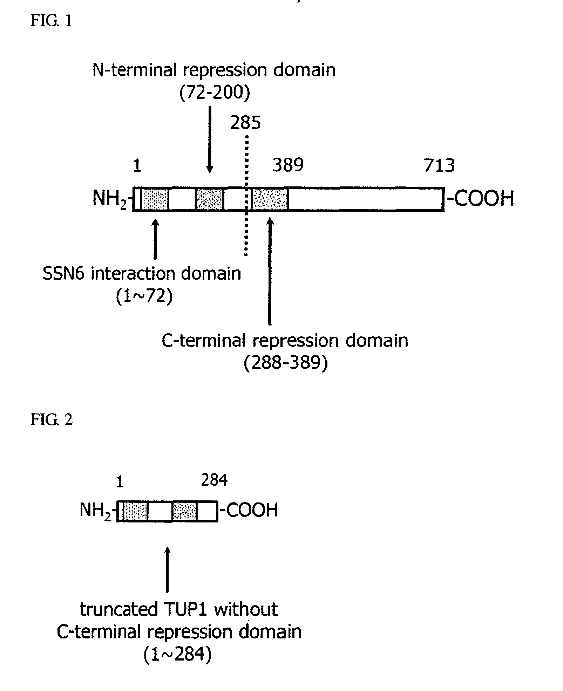

FIG. 1 is a schematic diagram of TUP1 protein, with the dotted line bisecting the sequence indicating the truncation point after amino acid 284 for the mutant described in Example 1;
FIG. 2 is a schematic diagram of the truncated TUP1 protein of Example 1 in which the C-terminal repression domain has been deleted;
FIG. 3 shows a method of screening genes resulting in increased galactose catabolism;
FIG. 4 is an enlarged genetic map of the region of yeast chromosome III containing the TUP1 gene (261594-263396);
FIG. 5 is a cleavage map of plasmid pRS424 used in Example 1 to construct the genomic library;
FIGS. 6 and 7 are graphs showing ethanol production resulting from culturing *S*. *cerevisiae* in minimal media containing a sugar mixture (containing 2% glucose and 2% galactose) as well as OD₆₀₀ and concentration of glucose and galactose remaining (expressed as g/L) as a function of time (FIG. 6: Wild-type strain; FIG. 7: truncated TUP1-overexpressing stain);
FIGS. 8 and 9 are graphs showing ethanol production resulting from culturing *S*. *cerevisiae* in minimal media containing 4% galactose as well as OD₆₀₀ and galactose (in g/L) remaining as a function of time (FIG. 8: Wild-type stain; FIG. 9: truncated TUP1-overexpressing stain);
FIGS. 10 and 11 are graphs showing ethanol production resulting from culturing *S*. *cerevisiae* in minimal media containing 10% galactose as well as OD₆₀₀ and the galactose remaining as a function of time (FIG. 10: Wild-type strain; FIG. 11: truncated TUP1-overexpressing stain); and
FIG. 12 is a graph showing ethanol production resulting from culturing four different *S*. *cerevisiae* strains (wild-type; wild-type TUP1 overexpressing; truncated TUP1 overexpressing; and TUP1 knockout) in minimal media containing 10% galactose as a function of time.

### DETAILED DESCRIPTION

Hereinafter, the inventive concept will now be described more fully with reference to exemplary embodiments and the accompanying drawings. However, it should be understood that the inventive concept is not limited to the described exemplary embodiments and may be embodied in various modifications and changes.

### 1. Recombinant Gene and Recombinant Protein encoded therein

A novel recombinant repressor gene is disclosed. The recombinant repressor gene is truncated such that all or a portion of a repression domain is deleted from the encoded protein. The wild-type repressor protein represses expression of a galactose-catabolizing gene.

Galactose is an aldohexose, molecular formula C₆H₁₂O₆, which is converted into galactose-1-phosphate and then glucose-1-phosphate in many organisms. In some microorganisms, e.g., yeast, the glucose-1-phosphate can be catabolized into ethanol by fermentation. Herein, the concentration of ethanol produced per galactose consumption time is denoted as the "volumetric productivity of ethanol".

Galactose-catabolizing genes in yeast, i.e., genes involved in galactose uptake and metabolism, include gal2, gal1, gal7, gal10, and gal5 (pgm1 or pgm2).

The term 'galactose catabolism' refers to the metabolic degradation of galactose. The level of galactose catabolism is often expressed as the 'galactose utilization rate'.

A gene encoding a protein comprising a repression domain ('repressor gene') may include tup1, gal4, gal3, gal80, gal6, mig1, and ssn6. By genetic manipulation of these repressor genes, expression of the galactose-catabolizing gene may not be suppressed. For example, expression of a galactose-catabolizing gene may be increased by deleting or modifying a repression domain that functions as an activation site of the repressor protein.

In one example, the repressor gene is the gene encoding TUP1 protein. In some embodiments, the TUP1 repressor gene is a yeast gene. Specifically, the TUP1 repressor gene can be from *S*. *cerevisiae.*

The TUP1 protein is known to function as a general transcriptional co-repressor in yeast. Among the genes which TUP1 regulates are genes involved in galactose catabolism. In particular, *Saccharomyces cerevisiae* TUP1 forms a complex with CYS8 (SSN6), which represses transcription of genes regulated by glucose, oxygen, and DNA damage.

While neither TUP1 nor CYS8(SSN6) binds directly to DNA, each functions as an element of a co-repressor through interaction with various DNA-binding proteins such as α 2, Mig1, Rox1, or al. For example, the TUP1 protein can bind to SSN6 protein thereby forming a complex that suppresses expression of a galactose-catabolizing gene, guided by the DNA binding protein Mig1.

Therefore, when a part of the repression domain in TUP1 is inactivated, for example by deletion, expression of the galactose-catabolizing genes may not be suppressed. For convenience, a TUP1 protein in which a part of the expression repression domain is deleted is called a "truncated TUP1 protein".

Accordingly, without being bound by theory, it is believed that when the gene encoding truncated TUP1 protein is overexpressed, the repression mechanism does not operate properly, resulting in expression of the galactose-catabolizing genes.

All or part of the repression domain of the repressor gene may be deleted. If at least two repression domains are present in the repressor gene, then only one or both of them may be deleted.

The repression domain may be a C-terminal repression domain. The truncated gene is a gene encoding TUP1 protein in which all or part of its C-terminal repression domain is deleted.

Herein, a "C-terminal domain" refers to a domain including the carboxylic acid terminus of the protein, corresponding in the gene to the amino acid sequence encoded by the nucleic acid sequence located at the 3' end of the protein-coding region of the gene.

The C-terminal domain of a repressor protein may include the final 300 amino acids counting from the C-terminus of the polypeptide, or the final 1/3 of the polypeptide counting from the C-terminus of the polypetide. The "C-terminal domain" of a polypeptide is not shorter than 3 amino acids and not longer than 350 amino acids and may include 5, 10, 20, 25, 50, 100, or 200 amino acids.

The C-terminal half of TUP1 protein contains six repeats of a 43-amino acid sequence motif rich in aspartate and tryptophan, known as WD-40 or β-transducin repeats. WD-40 repeats have been identified in many proteins and are known to play a role in protein--protein interactions.

Thus, the portion of the C-terminal repression domain deleted from the truncated TUP1 may include one or more WD-40 repeats.

It is disclosed that the truncated gene may encode TUP1 protein in which a portion of the C-terminal repression domain found at positions 288-389 in the amino-acid sequence is deleted. Herein, a gene encoding a truncated TUP1 protein in which a portion of positions 288-389 in the amino-acid sequence is deleted is referred to as a "truncated tup1" gene.

In one example, the truncated TUP1 protein can be truncated after amino-acid 284. The isolated polypeptide of such a truncated TUP1 protein has the amino acid sequence of SEQ ID NO:2. The truncated TUP1 protein with the amino acid sequence of SEQ ID NO:2 includes the SSN6 interaction domain and the N-terminal repression domain at amino acid positions 72 to 200. In another example, the recombinant TUP1 protein is encoded by the polynucleotide sequence of SEQ ID NO:1.

In another exemplary embodiment, a recombinant TUP1 protein in which a part of the C-terminal repression domain is truncated is provided. In this embodiment, the recombinant TUP1 protein has a partially deleted C-terminal repression domain, so that TUP1 becomes inactive in repressing expression of glucose-catabolizing genes, and instead permits expression of galactose-catabolizing genes.

Isolated polynucleotides encoding the recombinant repressor proteins are also provided.

"Isolated," when used to describe the various polypeptides or polynucleotides disclosed herein, means a polypeptide or polynucleotide that has been identified and separated and/or recovered from a component of its natural environment. The term also embraces recombinant polynucleotides and polypeptides and chemically synthesized polynucleotides and polypeptides .

In an embodiment, the isolated polynucleotide consists of a nucleotide sequence encoding a recombinant protein, the amino acid sequence of which consists of SEQ ID NO:2.

In another embodiment, the isolated polynucleotide consists of the nucleotide sequence of SEQ ID NO: 1.

### 2. Recombinant Vector and Recombinant microorganism

A recombinant vector including the isolated polynucleotide encoding a TUP1 protein in which all or part of one of the repression domains is deleted is provided. In an embodiment, the recombinant vector comprises an isolated polynucleotide encoding a truncated TUP1 protein having the amino acid sequence of SEQ ID NO:2.

The term "vector" refers to a nucleic acid molecule capable of transporting another nucleic acid to which it has been linked. Available vectors may include bacteria, plasmids, phages, cosmids, episomes, viruses, and insertable DNA fragments (fragments able to be inserted into a host cell genome by homologous recombination).

The term "plasmid" refers to a circular, extra-chromosomal, double-stranded DNA molecule typically capable of autonomous replication within a suitable host and into which a foreign DNA fragment can be inserted. Further, the term "virus vector" refers to a vector in which foreign DNA has been inserted into a virus genome for delivery into cells.

Herein, a vector directing expression of a gene encoding a target protein operably linked thereto is called an "expression vector." Generally, in recombinant DNA technology, a plasmid is used as an expression vector, and thus the term "plasmid" may be interchangeably used with the term "expression vector." However, it should be clear that expression vectors also include other types of vectors exhibiting the same function, for example, a virus vector.

The term "operably linked" refers to the association of nucleic acid sequences on a single nucleic acid fragment so that the function of one is regulated by the other. For example, a promoter is operably linked with a coding sequence when it is capable of regulating the expression of that coding sequence (i.e., that the coding sequence is under the transcriptional control of the promoter) or a ribosome binding site is operably linked to a coding sequence if it is positioned so as to facilitate translation. Coding sequences can be operably linked to regulatory sequences in a sense or antisense orientation.

In some embodiments, the expression vector can introduce and express a specific gene I in yeast, Examples of such expression vectors include vector II micron, pBM272, pBR322-6, pBR322-8, pCS19, pDW227, pDW229, pDW232, pEMBLYe23, pEMBLYe24, pEMBLYi21, pEMBLYi22, pEMBLYi32, pEMBLYr25, pFL2, pFL26, pFL34, pFL35, pFL36, pFL38, pFL39, pFL40, pFL44L, pFL44S, pFL45L, pFL45S, pFL46L, pFL46S, pFL59, pFL59+, pFL64-, pFL64+, pG6, pG63, pGAD10, pGAD424, pGBT9, pGK12, pJRD171, pKD1, pNKY2003, pNKY3, pNN414, pON163, pON3, pPM668, pRAJ275, pRS200, pRS303, pRS304, pRS305, pRS306, pRS313, pRS314, pRS315, pRS316, pRS403, pRS404, pRS405, pRS406, pRS413, pRS414, pRS415, pRS416, pRS423, pRS424, pRS425, pRS426, pRSS56, pSG424, pSKS104, pSKS105, pSKS106, pSZ62, pSZ62, pUC-URA3, pUT332, pYAC2, pYAC3, pYAC4, pYAC5, pYAC55, pYACneo, pYAC-RC, pYES2, pYESHisA, pYESHisB, pYESHisC, pYEUra3, rpSE937, YCp50, YCpGALO, YCpGAL1, YCplac111, YCplac22, YCplac33, YDp-H, YDp-K, YDp-L, YDp-U, YDp-W, YEp13, YEp213, YEp24, YEp351, YEp352, YEp353, YEp354, YEp355, YEp356, YEp356R, YEp357, YEp357R, YEp358, YEp358R, YEplac112, YEplac181, YEplac195, YIp30, YIp31, YIp351, YIp352, YIp353, YIp354, YIp355, YIp356, YIp356R, YIp357, YIp357R, YIp358, YIp358R, YIp5, YIplac128, YIplac204, YIplac211, YRp12, YRp17, YRp7, pAL19, paR3, pBG1, pDBlet, pDB248X, pEA500, pFL20, pIRT2, pIRT2U, pIRT2-CAN1, pJK148, pJK210, pON163, pNPT/ADE1-3, pSP1, pSP2, pSP3, pSP4, pUR18, pUR19, pZA57, pWH5, pART1, pCHY21, pEVP11, REP1 ,REP3, REP4, REP41, REP42, REP81, REP82, RIP, REP3X, REP4X, REP41X, REP81X, REP42X, REP82X, RIP3X/s, RIP4X/s, pYZ1N, pYZ41N, pYZ81N, pSLF101, pSLF102, pSLF104, pSM1/2, p2UG, pART1/N795, and pYGT.

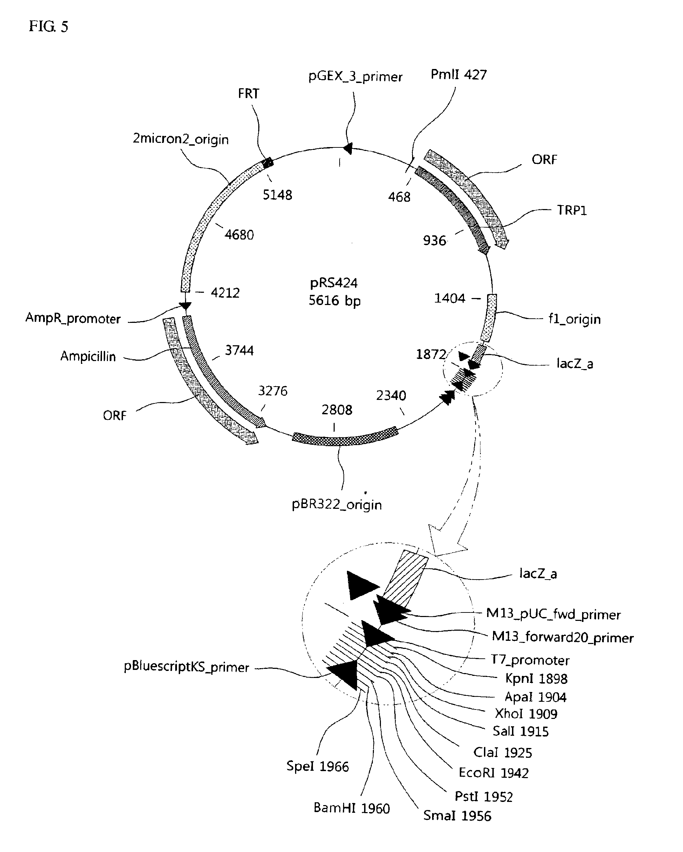

In some embodiments, the isolated polynucleotide is inserted into a multicopy plasmid to supply multiple copies of the inserted gene for high expression of the truncated TUP1 protein. Alternatively, the isolated polynucleotide is inserted into a low-copy plasmid containing a strong promoter to achieve high expression of the protein. In one example, the plasmid is plasmid pRS424, having the cleavage map shown in FIG. 5.

The vector may be introduced into a host cell, and produce a protein or peptide such as a fusion protein or peptide encoded by the genes mentioned above. In some cases, the vector may contain a promoter recognized by the host cell. By "promoter" is meant minimal sequence sufficient to direct transcription. Also included are those promoter elements which are sufficient to render promoter-dependent gene expression controllable for cell-type specific or inducible by external signals or agents; such elements may be located in the 5' or 3' regions of the gene. Both constitutive and inducible promoters are included (see e.g., Bitter et al. (1987) Methods in Enzymology 153: 516-544).

The promoter sequence may originate from a prokaryote, a eukaryote, or a virus. Yeast-compatible promoters include GAPDH, PGK, ADH, PHO5, GAL1 and GAL10. Selecting a yeast-compatible promoter with a suitable promoter activity for the desired level of expression of a gene in yeast is within the skill of the ordinary artisan.

The vector may have an additional expression control sequence. "Control sequences" refers to DNA sequences necessary for the expression of an operably linked coding sequence in a particular host organism. The control sequence may be a Shine-Dalgamo sequence. For example, the Shine-Dalgarno sequence can be from the replicase gene of phage MS-2 or from the cII gene of bacteriophage □. Moreover, the vector may have an appropriate marker to screen the transformed host cell. The transformation of a host may be accomplished by any one of a variety of techniques well known in the art.

A recombinant microorganism comprising the isolated polynucleotide encoding a truncated TUP1 protein is also disclosed. The recombinant microorganism comprises an isolated polynucleotide encoding the truncated TUP1 protein having the amino acid sequence of SEQ ID NO:2.

In an embodiment, the microorganism is a yeast.

In some embodiments, the yeast is selected from the genus *Saccharomyces,* the genus *Pachysolen,* the genus *Clavispora,* the genus *Kluyveromyces,* the genus *Debaryomyces,* the genus *Schwanniomyces,* the genus *Candida,* the genus *Pichia,* or the genus *Dekkera.*

Without being bound by theory, it is believed that in such a recombinant yeast strain, the repression activity of wild-type TUP1 protein is inhibited by the gene encoding the truncated TUP1 protein, and thus repression of expression of a galactose-catabolizing gene does not operate properly. As a result, expression of the galactose-catabolizing gene is stimulated, and then galactose may be rapidly converted to ethanol in a medium containing as the carbon source either a mixture of glucose and galactose or only galactose. Thus,the recombinant yeast strain can exhibit improved volumetric productivity of ethanol from a process of culturing the recombinant yeast strain in a galactose-containing medium.

The recombinant yeast may increase ethanol volumetric productivity at least about 30%, or at least about 50% over the ethanol volumetric productivity of the wild-type yeast.

The recombinant yeast can be produced by transforming the parent microorganism with a recombinant vector disclosed herein. Transformation of the yeast is conducted by any transformation method known to a person of ordinary skill in the art. For example, transformation of a yeast with the recombinant vector may be conducted by the method of Ito, H., et al. (J. Bacteriol. (1983) 153, 163-168).

In some embodiments in order to transform *S. cerevisiae* CEN.PK2-1D with a vector containing a heterogeneous gene, a spheroplast transformation kit (Bio 101, Vista, CA) is used. In such an embodiment, the transformed strain may be cultured in yeast synthetic complete (YSC) medium containing 20g/l of glucose, and then continuously cultured in YSC medium containing 4% galactose. Afterward, strains with an improved galactose utilization rate may be screened on 4% galactose-containing YSC solid medium.

In one embodiment, a recombinant yeast deposited as Accession No. KCTC 11387 BP is provided.

The deposited recombinant yeast determined by screening to have an excellent galactose utilization rate, was deposited under the name of *Saccharomyces cerevisiae* CEN.PK2-1D/pRS424-truncated TUP1 on September 4, 2008 to the Gene bank of the Korea Research Institute of Bioscience and Biotechnology (Yuseung-gu, Daejeon, Korea) with Accession No. KCTC 11387 BP.

### 3. Method of Producing Ethanol

A method of producing ethanol from a galactose-containing carbon source is also provided. In an embodiment, the method comprises culturing a recombinant yeast disclosed herein in a galactose-containing carbon source such that ethanol is produced. The method can further comprise recovering the ethanol.

Herein, when the gene is overexpressed, volumetric productivity of ethanol is increased.

The galactose-containing carbon source may contain a mixture of galactose and glucose, or only galactose. The mixed ratio of the galactose to glucose in the medium is not particularly limited, but the medium may contain at least about 4.0% galactose.

As shown below in the Examples, culturing the recombinant yeast that overexpresses the truncated TUP1 gene in a medium containing either a mixture of glucose and galactose, or only galactose, as a carbon source, yields greatly increased volumetric productivity of ethanol, compared to the parent microorganism.

The galactose-containing carbon source may be a hydrolysate of algae biomass.

The kind of algae is not particularly limited and may include red algae (e.g., Porphyra yezoensis Ueda), brown algae (e.g., the Laminariaceae family, Undaria pinnatifida and Hizikia fusiforme), and green algae (e.g., Enteromorpha genus).

The red algae may include *Gelidium amansii, Gracilaria verrucosa, Bangia atropurpurea, Porphyra suborbiculatca, Porphyra yezoensis, Galaxaura falcate, Scinaia japonica, Gelidium divaricatum, Gelidium pacificum, Lithophylum okamurae, Lithothammion cystocarpideum, Amphiroa anceps, Amphiroa beauvoisii, Corallina officinalis, Corallina pilulifera, Marginisporum aberrans, Carpopeltis prolifera, Grateloupia filicina, Grateloupia elliptica, Grateloupia lanceolanta, Grateloupia turtuturu, Phacelocarpus japonicus, Gloiopeltis furcata, Hypnea charoides, Hypnea japonitca, Hypnea saidana, Chondrus cripspus, Chondracanthus tenellus, Gracilaria textorii, Lomentaria catenata, Heterosiphonia japonica, Chondria crassicaulis,* and *Symphyocladia latiuscula.*

A method of producing ethanol from algae biomass may be conducted by any method known in the art. For example, ethanol may be produced from red algae biomass by direct saccharification which the red algae is directly saccharified, or by indirect saccharificationin which agar or cellulose is extracted from the red algae and then saccharified to obtain galactose or glucose. The saccharification may be performed by enzyme hydrolysis using galactocidase, or acid hydrolysis using a catalyst for acid hydrolysis. Then, ethanol may be produced by fermentation using any microorganism.

When ethanol is produced from algae biomass, which is abundant in nature, resource supply and demand can be stable and no pretreatment processes are necessary. Thus, very high production efficiency may be obtained.

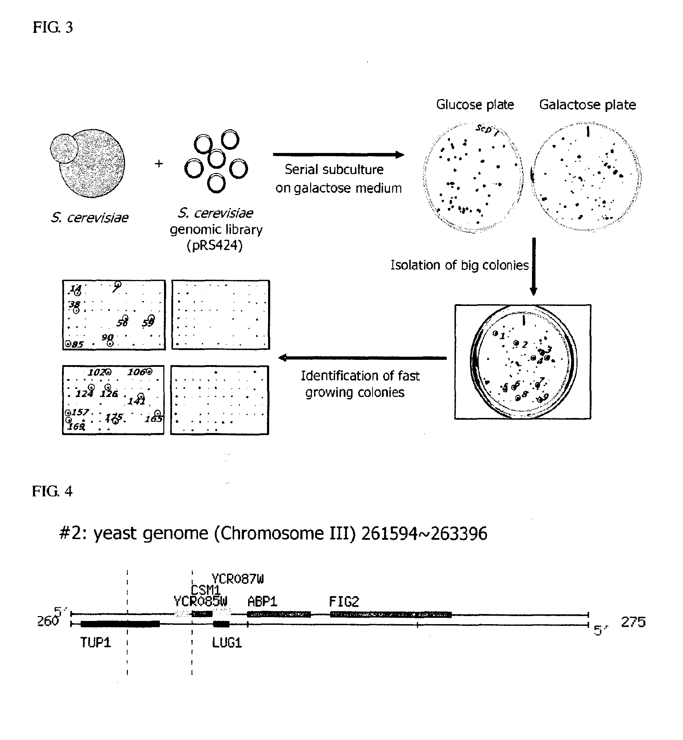

FIG. 3 illustrates a method of screening for genes yielding increased galactose utilization. The gene screening method will be described in detail with reference to FIG. 3.

Further disclosed is that in the method, the yeast may be *S*. *cerevisiae* CEN.PK2-1D, and the multi-copy plasmid may be pRS424.

Further disclosed is that in the method, the yeast genomic DNA library can be prepared by cutting *S*. *cerevisiae* CEN.PK2-1D genomic DNA with a restriction enzyme, ligating a yeast DNA fragment into the multi-copy plasmid (pRS424), and amplifying the recombinant plasmid in *E.coli.*

Further disclosed is that the transformed yeast library may be prepared by the method described by Ito et al. (J. bacteriol. (1983) 153, 163-168).

Then, the transformed yeast are selected for those with increased galactose utilization. Such colonies exhibit faster growth as evidenced by the formation of big colonies in serial subculture. Afterward, the inserted yeast genomic sequence in the selected transformed yeast is identified. The genomic sequence may be analyzed using a gel documentation (gel doc).

Also disclosed is that the gene screening method may further include: detecting the location of the inserted gene on the yeast genome by comparing the base sequence of the yeast genome with a predetermined length of genomic sequence present at each end of the gene inserted into the plasmid to identify the overexpressed gene; or re-transforming a yeast with the plasmid containing the identified gene to confirm an increase in galactose catabolism is caused by overexpression of the gene.

In one embodiment, the gene increasing galactose catabolism upon overexpression encodes a TUP1 protein in which at least a part of the C-terminal repression domain is deleted, as described above.

The following examples further illustrate the invention but, of course, should not be construed as in any way as limiting its scope.

### [Preparation Example 1]

The recombinant TUP1 gene was selected using the procedures schematically described in FIG. 3.

A yeast genomic DNA library was constructed by ligating size-selected *S*. *cerevisiae* genomic DNA fragments into a multi-copy plasmid (pRS424). Subsequently, competent *E. coli* cells were transformed with the recombinant multicopy plasmids to create the genomic library.

*S. cerevisiae* CEN.PK2-1D (MATalpha; ura3-52; trp1-289; leu2-3_112; his3 D1; MAL2-8C; SUC2) was then transformed with the *S*. *cerevisiae* genomic library to construct a transformed yeast library in which all the *S*. *cerevisiae* genes are overexpressed.

The transformed yeast library was cultured in a medium containing only galactose as a carbon source. The cultured transformed yeast were then screened by serial subculturing to select those yeast strains exhibiting increased galactose utilization. Transformed yeast exhibiting increased galactose utilization will grow more quickly on a medium containing only galactose as a carbon source than yeast with normal galactose utilization and therefore will form big colonies. Plasmids were isolated from the yeast exhibiting increased galactose utilization to permit determination of the yeast genomic sequence inserted into the plasmid by sequencing.

The location of the yeast genomic sequence insert on the yeast genome was determined by comparing the base sequence of the yeast genome with a predetermined length of genomic sequence present at each end of a gene inserted into the plasmid to identify the gene;

A plasmid with an inserted gene encoding TUP1 protein (3rd chromosome 261594-263396) in which the part of the C-terminal repression domain beyond amino acid 284 (the polypeptide of SEQ ID NO:2) was identified in general accordance with the above procedures.

*S .cerevisiae* CEN.PK2-1D was then transformed with the multi-copy plasmid encoding the truncated TUP1 protein containing only amino acids 1-284 using a spheroplast transformation kit (Bio 101, Vista, CA) in order to confirm that overexpression of the gene resulted in an increase in galactose catabolism. The transformed strain was cultured in yeast synthetic complete (YSC) medium containing 20g/l of glucose, and as much amino acids and nucleotides as needed were provided.

### [Preparative Example 2]

A strain of *Pichia sp* is transformed with a multi-copy plasmid encoding the truncated TUP1 protein. The transformed strain is cultured in YSC medium containing 20g/l of glucose, and other necessary nutrients.

### [Preparative Example 3]

A strain of *Kluyveromyces sp* is transformed with a multi-copy plasmid encoding the truncated TUP1 protein. The transformed strain is cultured in YSC medium containing 20g/l of glucose, and other necessary nutrients.

### [Example 1]

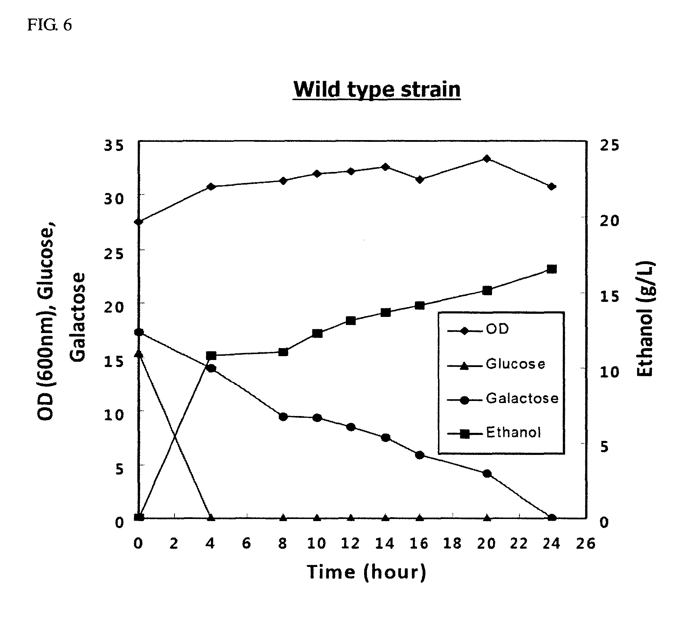

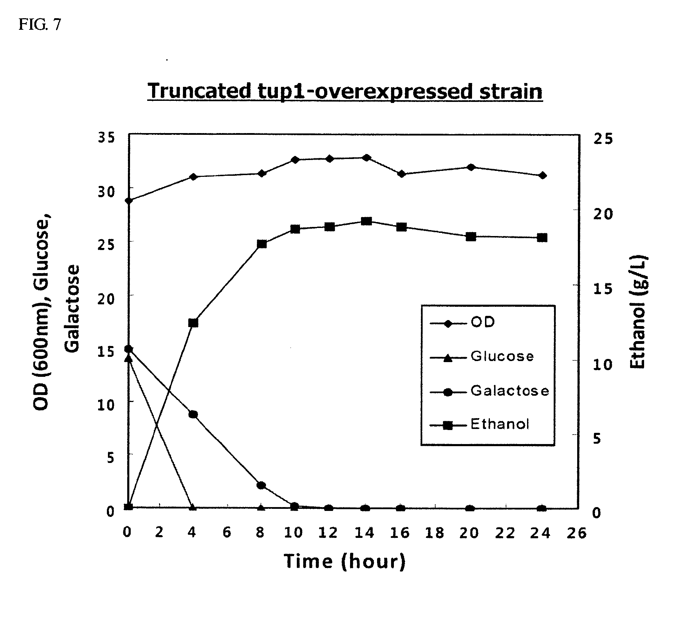

A culture of the wild type yeast strain (*wild type*) containing an empty vector and a culture of the recombinant yeast strain transformed with the gene encoding truncated TUP1 protein (*truncated tup1*) prepared in Preparation Example 1 were inoculated to achieve an initial OD₆₀₀ = 25 and then cultured in minimal medium containing a mixture of 2% each of glucose and galactose. Subsequently, galactose utilization and volumetric productivity of ethanol of each culture were determined. The results are shown in FIGS. 6 and 7, for the wild-type strain and the recombinant strain, respectively, and in Table 1.

**TABLE 1**

| | **Wild type** | **Truncated Tup1** |
|---|---|---|
| **Galactose consumption time** | 24 h | 10 h |
| **Ethanol concentration** | 16.5 g/l | 18.9 g/l |
| **Volumetric productivity** | 0.69 g/l h | 1.90 g/l h |

The results in Figs. 6 and 7 and in Table 1 show that the galactose utilization rate is significantly increased in the recombinant yeast strain transformed with the gene encoding truncated TUP1 protein, compared to the wild type strain.

Particularly, while the time to consume 2% galactose by the recombinant yeast strain transformed with the gene encoding truncated TUP1 protein was about 10 hours, the time for the wild type strains was about 24 hours. In addition, while the final ethanol concentration produced by the yeast strain transformed with the gene encoding truncated TUP1 protein was 18.9g/L, that produced by the wild type strains was 16.5g/L.

### [Example 2]

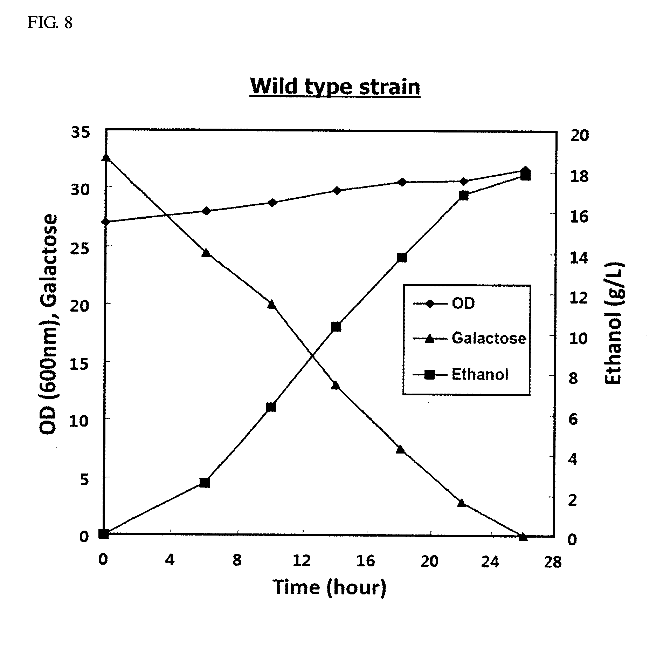

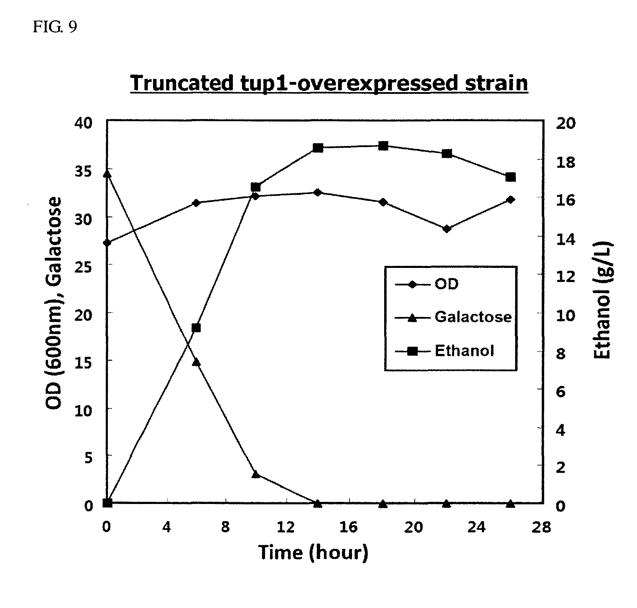

The wild type yeast strain (*wild type*) was compared to the recombinant yeast stain transformed with the gene encoding truncated TUP1 protein (*truncated TUP1*) prepared in Preparation Example 1 with respect to galactose utilization and volumetric productivity of ethanol by the same method as described in Example 1, except that the yeast strains were cultured in minimal medium containing only 4% galactose as a carbon source. The results are shown in FIGS. 8 and 9, for wild-type and recombinant yeast strains, respectively, and Table 2.

**TABLE 2**

| | **Wild type** | **Truncated tup1** |
|---|---|---|
| **Galactose consumption time** | 26h | 14h |
| **Ethanol concentration** | 17.8 g/l | 18.7 g/l |
| **Volumetric productivity** | 0.68 g/lh | 1.33g/lh |

The results show that the galactose utilization rate is higher in the recombinant yeast strain than in the wild type strain. Even with the same initial cell density (OD₆₀₀), the recombinant yeast strain consumed all galactose in about 14 hours, and produced 18.7g/L of ethanol. In contrast, the wild type strain produced 17.8g/L of ethanol and required 26 hours to consume all galactose. This indicates that the volumetric productivity of ethanol increases from 0.68g/L per hour determined for the wild-type strain to 1.33g/L per hour for the recombinant strain. That is, the volumetric productivity of ethanol increases by 95%, when the yeast strain was transformed with the gene encoding truncated TUP1 protein.

### [Example 3]

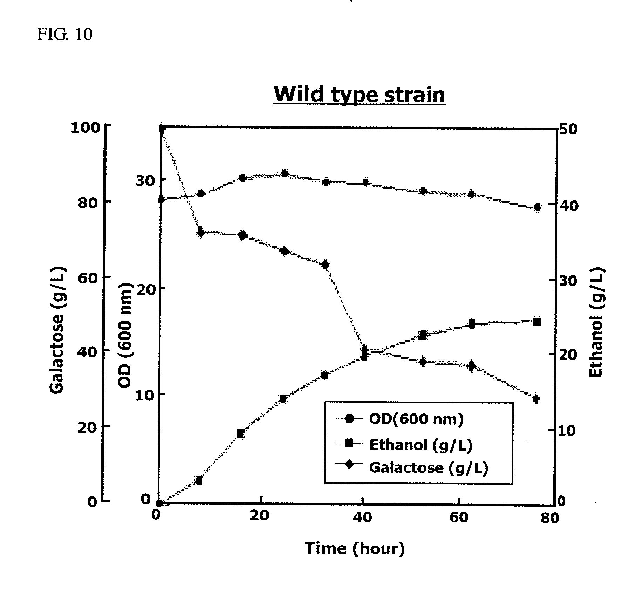

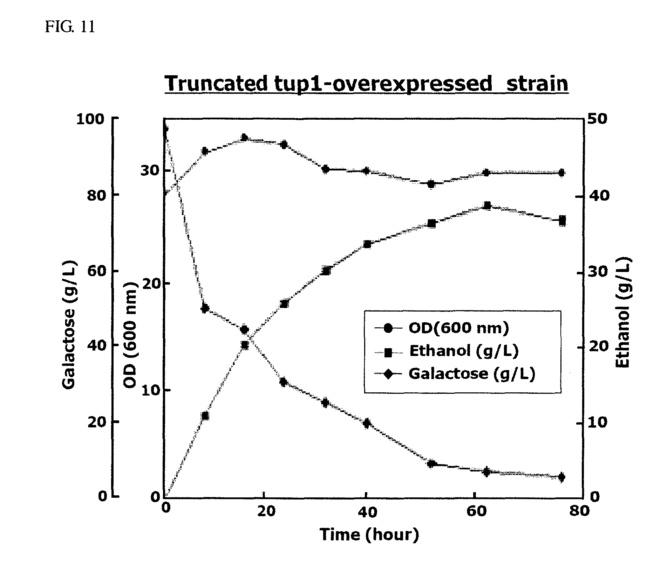

The wild type yeast strain (*wild type*) was compared to the recombinant yeast strain transformed with the gene encoding truncated TUP1 protein (*truncated tup1*) prepared in Preparation Example 1 with respect to galactose utilization rate and ethanol volume productivity by the same method as described in Example 1, except that the yeast strains were cultured in minimal medium containing only 10% galactose as a carbon source for 80 hours. The results are shown in FIGS. 10 and 11, for wild-type and recombinant yeast strains, respectively, and Table 3.

**TABLE 3**

| | **Wild type** | **Truncated tup1** |
|---|---|---|
| **Galactose consumption time** | 80h | 80h |
| **Ethanol concentration** | 25 g/l | 38 g/l |
| **Volumetric productivity** | 0.31 g/lh | 0.48 g/lh |

The results show that galactose utilization is higher in the recombinant yeast strain than in the wild type strain. Even with the same initial cell density, the recombinant yeast strain left only 5g/L of galactose after an 80-hour fermentation and produced 38g/L of ethanol. In contrast, the wild type strain left 23 g/L of galactose after fermentation for 80 hours and produced 25g/L of ethanol.

### [Example 4]

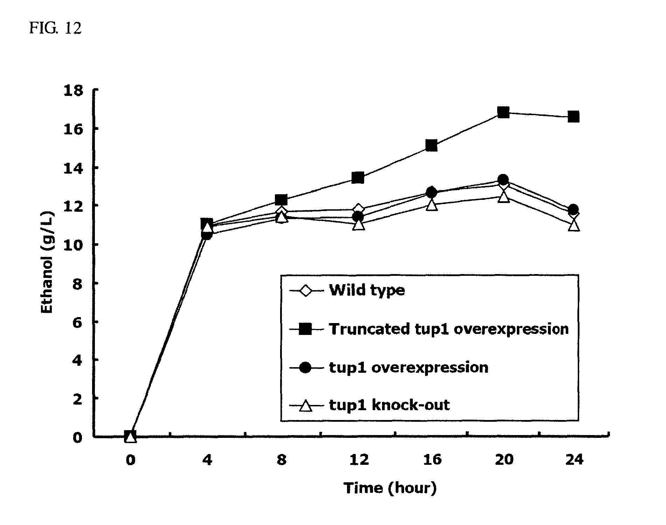

Volumetric productivity of ethanol was determined for a 1L culture of each of the following yeast strains: the yeast strain prepared in Preparative Example 1, overexpressing the gene encoding truncated TUP1 protein (truncated *tup1* overexpression); a wild-type yeast strain (wt control), a yeast strain overexpressing the wild-type TUP1-gene (wt tup 1 overexpression) and a TUP1-knock-out yeast strain (tup1 knockout). Each strain was cultured in minimal medium containing a mixture of 2% each glucose and galactose, and the volumetric productivity of ethanol was determined. The results are shown in FIG. 12, and Table 4.

**TABLE 4**

| **Time (h)** | **wt control** | **truncated tup1 overexpression** | **wt tup 1 overexpression** | **tup1 knockout** |
|---|---|---|---|---|
| **0** | 0 | 0 | 0 | 0 |
| **4** | 10.9586 | 11.024 | 10.4684 | 10.8903 |
| **8** | 11.665 | 12.2271 | 11.3107 | 11.3987 |
| **12** | 11.7704 | 13.4094 | 11.3433 | 11.0397 |
| **16** | 12.691 | 15.0325 | 12.6048 | 12.0053 |
| **20** | 13.0141 | 16.7613 | 13.29 | 12.4637 |
| **24** | 11.5268 | 16.5114 | 11.75 | 10.9448 |

The results show that volumetric productivity of ethanol by the the wild type yeast strain, the yeast strain overexpressin wild-type TUP1, and the TUP1-knock-out yeast strain was similar in the initial presence of 2%galactose/2%glucose. However, the yeast strain overexpressing the gene encoding truncated TUP1 protein produced 16.5g/L of ethanol in the 24-hour fermentation. Accordingly, volumetric productivity of ethanol was higher for the yeast strain overexpressing the gene encoding truncated TUP1 protein than for the wild-type control yeast strain.

The terminology used herein is for the purpose of describing particular embodiments only and is not intended to be limiting of the invention. The terms "a" and "an" do not denote a limitation of quantity, but rather denote the presence of at least one of the referenced item. The term "or" means "and/or". The terms "comprising", "having", "including", and "containing" are to be construed as open-ended terms (i.e., meaning "including, but not limited to").

Recitation of ranges of values are merely intended to serve as a shorthand method of referring individually to each separate value falling within the range, unless otherwise indicated herein, and each separate value is incorporated into the specification as if it were individually recited herein. The endpoints of all ranges are included within the range and independently combinable.
## Claims

1. An isolated polynucleotide consisting of a nucleotide sequence encoding a polypeptide, the amino acid sequence of which consists of SEQ ID NO:2.

2. The isolated polynucleotide of claim 1, wherein the nucleotide sequence consists of SEQ ID NO:1.

3. An isolated polypeptide, the amino acid sequence of which consists of SEQ ID NO:2.

4. A recombinant vector comprising the isolated polynucleotide of claim 1.

5. The recombinant vector of claim 4, which is a plasmid.

6. The recombinant vector of claim 4, wherein the plasmid comprises pRS424.

7. The recombinant vector of claim 4, wherein the isolated polynucleotide is operably linked to an expression control sequence.

8. The recombinant vector of claim 4, wherein the isolated polynucleotide consists of SEQ ID NO:1.

9. A recombinant microorganism comprising the isolated polynucleotide of claim 1, wherein the recombinant microorganism is a yeast.

10. The recombinant microorganism of claim 9, wherein the isolated polynucleotide is operably linked to an expression control sequence.

11. The recombinant microorganism of claim 9, comprising a vector comprising the isolated polynucleotide.

12. The recombinant microorganism of claim 9, wherein the isolated polynucleotide consists of SEQ ID NO:1.

13. The recombinant microorganism of claim 9, wherein the yeast is selected from yeasts of the genus *Saccharomyces,* yeasts of the genus *Pachysole,* yeasts of the genus *Clavispora,* yeasts of the genus *Kluyveromyces,* yeasts of the genus *Debaryomyces,* yeasts of the genus *Schwanniomyces,* yeasts of the genus *Candida,* yeasts of the genus *Pichia,* and yeasts of the genus *Dekkera.*

14. The recombinant microorganism of claim 13, wherein the yeast is *Saccharomyces cerevisiae* CEN.PK2-1D/pRS424-truncated TUP1 (Accession No. KCTC 11387 BP).

15. A method of producing ethanol from a galactose-containing carbon source comprising culturing the recombinant microorganism of claim 9 in a galactose-containing carbon source such that ethanol is produced, wherein production of ethanol is increased by overexpression of the isolated polynucleotide.

16. The method of claim 15, wherein ethanol production is increased by at least about 30% compared to ethanol production during culturing of the microorganism without overexpression of the isolated polynucleotide.

17. The method of claim 15, wherein the galactose-containing carbon source contains only galactose, or a mixture of glucose and galactose.

18. The method of claim 15, wherein the galactose-containing carbon source contains at least about 4% of galactose.

19. The method of claim 15, further comprising recovering the ethanol.

20. A method of producing a recombinant yeast by transforming a yeast with the recombinant vector of claim 4.

21. The method of claim 20, wherein catabolism of galactose by the transformed yeast is greater than by the wildtype yeast.

22. The method of claim 20, wherein ethanol production from a culture with galactose as a carbon source is greater for the transformed yeast than for the wildtype yeast.

23. The method of claim 20, wherein the yeast is selected from yeasts of the genus *Saccharomyces,* yeasts of the genus *Pachysole,* yeasts of the genus *Clavispora,* yeasts of the genus *Kluyveromyces,* yeasts of the genus *Debaryomyces,* yeasts of the genus *Schwanniomyces,* yeasts of the genus *Candida,* or yeasts of the genus *Pichia,* and yeasts of the genus *Dekkera.*

24. The method of claim 23, wherein the yeast is of the genus Saccharomyces.

25. The method of claim 20, wherein the isolated polynucleotide consists of SEQ ID NO:1.

## Patentansprüche

1. Isoliertes Polynukleotid, das aus einer Nukleotidsequenz besteht, die ein Polypeptid mit der Aminosäuresequenz bestehend aus der Sequenz der SEQ ID Nr. 2 kodiert.

2. Isoliertes Polynukleotid gemäß Anspruch 1, wobei die Nukleotidsequenz aus der Sequenz der SEQ ID Nr. 1 besteht.

3. Isoliertes Polypeptid, dessen Aminosäuresequenz aus der Sequenz der SEQ ID Nr. 2 besteht.

4. Rekombinanter Vektor umfassend das isolierte Polynukleotid gemäß Anspruch 1.

5. Rekombinanter Vektor gemäß Anspruch 4, der ein Plasmid ist.

6. Rekombinanter Vektor gemäß Anspruch 4, wobei das Plasmid pRS424 umfasst.

7. Rekombinanter Vektor gemäß Anspruch 4, wobei das isolierte Polynukleotid in operativer Weise mit einer Expressionskontrollsequenz verknüpft ist.

8. Rekombinanter Vektor gemäß Anspruch 4, wobei das isolierte Polynukleotid aus der Sequenz der SEQ ID Nr. 1 besteht.

9. Rekombinanter Mikroorganismus, der das isolierte Polynukleotid gemäß Anspruch 1 umfasst, wobei der rekombinante Mikroorganismus eine Hefe ist.

10. Rekombinanter Mikroorganismus gemäß Anspruch 9, wobei das isolierte Polynukleotid in operativer Weise mit einer Expressionskontrollsequenz verknüpft ist.

11. Rekombinanter Mikroorganismus gemäß Anspruch 9 umfassend einen Vektor, der das isolierte Polynukleotid umfasst.

12. Rekombinanter Mikroorganismus gemäß Anspruch 9, wobei das isolierte Polynukleotid aus der Sequenz der SEQ ID Nr. 1 besteht.

13. Rekombinanter Mikroorganismus gemäß Anspruch 9, wobei die Hefe ausgewählt ist aus Hefen der Gattung *Saccharomyces,* Hefen der Gattung *Pachysolen,* Hefen der Gattung *Clavispora,* Hefen der Gattung *Kluyveromyces,* Hefen der Gattung *Debaryomyces,* Hefen der Gattung *Schwanniomyces,* Hefen der Gattung *Candida,* Hefen der Gattung *Pichia* und Hefen der Gattung *Dekkera.*

14. Rekombinanter Mikroorganismus gemäß Anspruch 13, wobei die Hefe *Saccharomyces cerevisiae* CEN.PK2-1D/pRS424-trunkiertes TUP1 (Hinterlegungsnummer KCTC 11387 BP) ist.

15. Verfahren zum Herstellen von Ethanol aus einer Galaktose-enthaltenden Kohlenstoffquelle umfassend das Kultivieren des rekombinanten Mikroorganismus gemäß Anspruch 9 in einer Galaktose-enthaltenden Kohlenstoffquelle derart, dass Ethanol produziert wird, wobei die Produktion von Ethanol aufgrund der Überexpression des isolierten Polynukleotides gesteigert ist.

16. Verfahren gemäß Anspruch 15, wobei die Ethanolproduktion um wenigstens 30%, verglichen mit der Ethanolproduktion während des Kultivierens von einem Mikroorganismus ohne Überexpression des isolierten Polynukleotides, gesteigert ist.

17. Verfahren gemäß Anspruch 15, wobei die Galaktose enthaltende Kohlenstoffquelle nur Galaktose oder ein Gemisch aus Glukose und Galaktose enthält.

18. Verfahren gemäß Anspruch 15, wobei die Galaktose-enthaltende Kohlenstoffquelle wenigstens 4% Galaktose enthält.

19. Verfahren gemäß Anspruch 15, das ferner die Rückgewinnung von Ethanol umfasst.

20. Verfahren zum Herstellen einer rekombinanten Hefe durch Transformation einer Hefe mit dem rekombinanten Vektor gemäß Anspruch 4.

21. Verfahren gemäß Anspruch 20, wobei der Katabolismus von Galaktose in der transformierten Hefe größer ist als im Wildtyp der Hefe.

22. Verfahren gemäß Anspruch 20, wobei die Ethanolproduktion aus einer Kultur mit Galaktose als Kohlenstoffquelle bei der transformierten Hefe größer ist als beim Wildtyp der Hefe.

23. Verfahren gemäß Anspruch 20, wobei die Hefe ausgewählt ist aus Hefen der Gattung *Saccharomyces,* Hefen der Gattung *Pachysolen,* Hefen der Gattung *Clavispora,* Hefen der Gattung *Kluyveromyces,* Hefen der Gattung *Debaryomyces,* Hefen der Gattung *Schwanniomyces,* Hefen der Gattung *Candida,* Hefen der Gattung *Pichia* und Hefen der Gattung *Dekkera.*

24. Verfahren gemäß Anspruch 23, wobei die Hefe von der Gattung Saccharomyces ist.

25. Verfahren gemäß Anspruch 23, wobei das isolierte Polynukleotid aus der Sequenz gemäß SEQ ID Nr. 1 besteht.

## Revendications

1. Polynucléotide isolé consistant en une séquence de nucléotides codant un polypeptide dont la séquence d'acides aminés est la séquence SEQ ID N°2.

2. Polynucléotide isolé selon la revendication 1, dans lequel la séquence de nucléotides est la séquence SEQ ID N°1.

3. Polypeptide isolé dont la séquence d'acides aminés est la séquence SEQ ID N°2.

4. Vecteur recombinant comprenant le polynucléotide isolé selon la revendication 1.

5. Vecteur recombinant selon la revendication 4, dans lequel le vecteur est un plasmide.

6. Vecteur recombinant selon la revendication 4, dans lequel le plasmide comprend pRS424.

7. Vecteur recombinant selon la revendication 4, dans lequel le polynucléotide isolé est fonctionnellement lié à une séquence de contrôle d'expression.

8. Vecteur recombinant selon la revendication 4, dans lequel le polynucléotide isolé est la séquence SEQ ID N°1.

9. Microorganisme recombinant comprenant le polynucléotide isolé selon la revendication 1, dans lequel le microorganisme recombinant est une levure.

10. Microorganisme recombinant selon la revendication 9, dans lequel le polynucléotide isolé est fonctionnellement lié à une séquence de contrôle d'expression.

11. Microorganisme recombinant selon la revendication 9, comprenant un vecteur qui comporte le polynucléotide isolé.

12. Microorganisme recombinant selon la revendication 9, dans lequel le polynucléotide isolé est la séquence SEQ ID N°1.

13. Microorganisme recombinant selon la revendication 9, dans lequel la levure est sélectionnée parmi les levures du genre *Saccharomyces,* les levures du genre *Pachysolen,* les levures du genre *Clavispora,* les levures du genre *Kluyveromyces,* les levures du genre *Debaryomyces,* les levures du genre *Schwanniomyces,* les levures du genre *Candida,* les levures du genre *Pichia* et les levures du genre *Dekkera.*

14. Microorganisme recombinant selon la revendication 13, dans lequel la levure est *Saccharomyces cerevisiae* CEN.PK2-1D TUP1 à pRS424 tronqué (numéro d'ordre KCTC 11387 BP).

15. Procédé de production d'éthanol à partir d'une source de carbone contenant du galactose, comprenant la culture du microorganisme recombinant selon la revendication 9 dans une source de carbone contenant du galactose de manière à produire de l'éthanol, dans lequel la production d'éthanol est augmentée par surexpression du polynucléotide isolé.

16. Procédé selon la revendication 15, dans lequel la production d'éthanol est augmentée d'au moins environ 30 % par rapport à la production d'éthanol durant la culture du microorganisme sans surexpression du polynucléotide isolé.

17. Procédé selon la revendication 15, dans lequel la source de carbone contenant du galactose contient uniquement du galactose ou un mélange de glucose et de galactose.

18. Procédé selon la revendication 15, dans lequel la source de carbone contenant du galactose contient au moins environ 4 % de galactose.

19. Procédé selon la revendication 15, comprenant en outre la récupération de l'éthanol.

20. Procédé de production d'une levure recombinante en transformant une levure avec le vecteur recombinant selon la revendication 4.

21. Procédé selon la revendication 20, dans lequel le catabolisme du galactose par la levure transformée est supérieur à celui de la levure sauvage.

22. Procédé selon la revendication 20, dans lequel la production d'éthanol avec du galactose comme source de carbone d'une culture de levure transformée est supérieure à celle d'une culture de levure sauvage.

23. Procédé selon la revendication 20, dans lequel la levure est sélectionnée parmi les levures du genre *Saccharomyces,* les levures du genre *Pachysolen,* les levures du genre *Clavispora,* les levures du genre *Kluyveromyces*, les levures du genre *Debaryomyces,* les levures du genre *Schwanniomyces,* les levures du genre *Candida,* les levures du genre *Pichia* et les levures du genre *Dekkera.*

24. Procédé selon la revendication 23, dans lequel la levure est du genre *Saccharomyces.*

25. Procédé selon la revendication 20, dans lequel le polynucléotide isolé est la séquence SEQ ID N°1.


<110> SAMSUNG ELECTRONICS CO., LTD
<120> Nucleic Acids and constructs for Increasing Galactose Catabolism and Methods Therefor
<130> EP64817FZpau
<140> not yet assigned
   <141> herewith
<150> KR 10-2008-107277
   <151> 2008-10-30
<160> 2
<170> KopatentIn 1.71
<210> 1
   <211> 855
   <212> DNA
   <213> Artificial Sequence
<220>
   <223> truncated tup1 gene
<400> 1
   atgactgcca gcgtttcgaa tacgcagaat aagctgaatg agcttctcga tgccatcaga     60
   caggagtttc tccaagtctc acaagaggca aatacctacc gtcttcaaaa ccaaaaggat    120
   tacgatttca aaatgaacca gcagctggct gagatgcagc agataagaaa caccgtctac    180
   gaactggaac taactcacag gaaaatgaag gacgcgtacg aagaagagat caagcacttg    240
   aaactagggc tggagcaaag agaccatcaa attgcatctt tgaccgtcca gcaacagcgg    300
   caacagcaac agcagcaaca ggtccagcag catttacaac agcaacagca gcagctagcc    360
   gctgcatctg catctgttcc agttgcgcaa caaccaccgg ctactacttc ggccaccgcc    420
   actccagcag caaacacaac tactggttcg ccatcggcct tcccagtaca agctagccgt    480
   cctaatctgg ttggctcaca gttgcctacc accactttgc ctgtggtgtc ctcaaacgcc    540
   caacaacaac taccacaaca gcaactgcaa cagcagcaac ttcaacaaca gcaaccacct    600
   ccccaggttt ccgtggcacc attgagtaac acagccatca acggatctcc tacttctaaa    660
   gagaccacta ctttaccctc tgtcaaggca cctgaatcta cgttgaaaga aactgaaccg    720
   gaaaataata atacctcgaa gataaatgac accggatccg ccaccacggc caccactacc    780
   accgcaactg aaactgaaat caaacctaag gaggaagacg ccaccccggc tagtttgcac    840
   caggatcact actta                                                     855
<210> 2
   <211> 284
   <212> PRT
   <213> Artificial Sequence
<220>
   <223> trucated TUP1 protein
<400> 2
   Met Thr Ala Ser Val Ser Asn Thr Gln Asn Lys Leu Asn Glu Leu Leu
   1               5                   10                  15
   Asp Ala Ile Arg Gln Glu Phe Leu Gln Val Ser Gln Glu Ala Asn Thr
               20                  25                  30
   Tyr Arg Leu Gln Asn Gln Lys Asp Tyr Asp Phe Lys Met Asn Gln Gln
           35                  40                  45
   Leu Ala Glu Met Gln Gln Ile Arg Asn Thr Val Tyr Glu Leu Glu Leu
       50                  55                  60
   Thr His Arg Lys Met Lys Asp Ala Tyr Glu Glu Glu Ile Lys His Leu
   65                  70                  75                      80
   Lys Leu Gly Leu Glu Gln Arg Asp His Gln Ile Ala Ser Leu Thr Val
                   85                  90                  95
   Gln Gln Gln Arg Gln Gln Gln Gln Gln Gln Gln Val Gln Gln His Leu
               100                 105                 110
   Gln Gln Gln Gln Gln Gln Leu Ala Ala Ala Ser Ala Ser Val Pro Val
           115                 120                 125
   Ala Gln Gln Pro Pro Ala Thr Thr Ser Ala Thr Ala Thr Pro Ala Ala
       130                 135                 140
   Asn Thr Thr Thr Gly Ser Pro Ser Ala Phe Pro Val Gln Ala Ser Arg
   145                 150                 155                     160
   Pro Asn Leu Val Gly Ser Gln Leu Pro Thr Thr Thr Leu Pro Val Val
                   165                 170                 175
   Ser Ser Asn Ala Gln Gln Gln Leu Pro Gln Gln Gln Leu Gln Gln Gln
               180                 185                 190
   Gln Leu Gln Gln Gln Gln Pro Pro Pro Gln Val Ser Val Ala Pro Leu
           195                 200                 205
   Ser Asn Thr Ala Ile Asn Gly Ser Pro Thr Ser Lys Glu Thr Thr Thr
       210                 215                 220
   Leu Pro Ser Val Lys Ala Pro Glu Ser Thr Leu Lys Glu Thr Glu Pro
   225                 230                 235                     240
   Glu Asn Asn Asn Thr Ser Lys Ile Asn Asp Thr Gly Ser Ala Thr Thr
                   245                 250                 255
   Ala Thr Thr Thr Thr Ala Thr Glu Thr Glu Ile Lys Pro Lys Glu Glu
               260                 265                 270
   Asp Ala Thr Pro Ala Ser Leu His Gln Asp His Tyr
           275                 280